# EUROPÄISCHE PATENTSCHRIFT

(11) **EP 0 749 965 B1**
(45) Veröffentlichungstag und Bekanntmachung des Hinweises auf die Patenterteilung: **24.11.1999**
(21) Anmeldenummer: 96109720.1
(22) Anmeldetag: 18.06.1996
(51) Int. Cl.: C07D 249/08, C07D 249/12, C07C 257/22

(54) **Verfahren zur Herstellung von 1,2,4-Triazoliumsalzen und 1,2,4-Triazolinen**
Process for the preparation of 1,2,4-triazolium salts and 1,2,4-triazoles
Procédé pour la préparation des sels de 1,2,4-triazolium et 1,2,4-triazoles

(30) Priorität: 22.06.1995 DE 19522715
(43) Veröffentlichungstag der Anmeldung: 27.12.1996
(73) Patentinhaber: BASF AKTIENGESELLSCHAFT, 67056 Ludwigshafen (DE)
(72) Erfinder: Schneider, Regina, Dr., 67136 Fussgönheim (DE); Melder, Johann-Peter, Dr., 67141 Neuhofen (DE); Teles, Joaquim Henrique, 67059 Ludwigshafen (DE); Gröning, Carsten, Dr., 68259 Mannheim (DE); Ebel, Klaus, Dr., 68623 Lampertheim (DE)

(56) Entgegenhaltungen:
- GB-A- 1 118 426
- J AMER CHEM SOC, Bd. 75, 1953, Seite 1471 XP000602283 M.R.ATKINSON ET AL: "Synthesis of 1,3-diphenyl-1,2,4-triazole"
- CHEM REVIEWS, Bd. 70, 1970, Seiten 151-170, XP000600407 D.G.NEILSON ET AL: "The chemistry of amidrazones"
- J CHEM SOC PERKIN TRANS I, 1987, Seiten 1153-1158, XP000601625 J.C.BRINDLEY ET AL: "Intramolecular reaction between nitro and carbodiimide groups; N' substituted N-acyl and N-imidoyl-thioureas"

## Beschreibung

Die vorliegende Erfindung betrifft ein neues Verfahren zur Herstellung von 1,2,4-Triazoliumsalzen und 1,2,4-Triazolinen der allgemeinen Formel Ia bzw. Ib 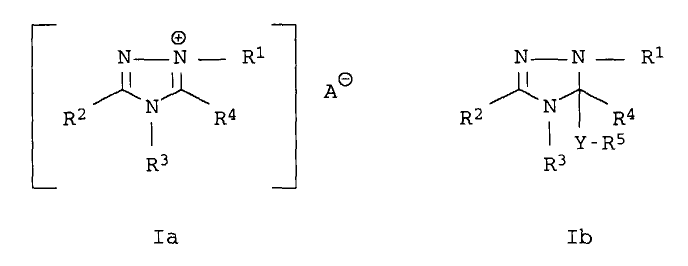 in der die Substituenten folgende Bedeutung haben:
- R¹ R² R³, R⁴, R⁵: gesättigte und ungesättigte aliphatische Reste mit 1 bis 30 C-Atomen, gesättigte und ungesättigte cycloaliphatische Reste mit 5 bis 7 Ringgliedern, araliphatische Reste mit 7 bis 30 C-Atomen, iso- und heterocyclische aromatische Reste, wobei R² und R³ zu einem 5 bis 8-gliedrigen Ring verbunden sein können und alle Reste R¹ bis R⁵ ihrerseits Fluor, Chlor, Brom, Cyan, Nitro und Hydroxyl als Substituenten tragen und durch Heteroatome unterbrochen sein können
- A: das Äquivalent eines Anions
- Y: Sauerstoff oder Schwefel
Außerdem betrifft die Erfindung ein neues Verfahren zur Herstellung der Amidrazone II.

Zur Herstellung von Triazoliumsalzen sind verschiedene Synthesewege bekannt, z.B. ausgehend von 1,3,4-trisubstituierten 1,2,4-Triazoliumsalzen aus den entsprechenden Triazolthionen durch oxidative Entschwefelung mit Salpetersäure oder Wasserstoffperoxid [R. Walentowski, H.W. Wanzlick, Z. Naturforsch. B, 25, 1421 (1970); H.G.O. Becker et al., J. Prakt. Chem. 330, 325 (1988)] oder ausgehend von Triazoliumsalzen durch Umsetzung von Iminen oder stickstoffhaltigen Heterocyclen (z.B. Pyridin) mit Alkoxydiazeniumsalzen [S. Hünig et al., Chem. Ber. 102, 3159 (1969); GB-A 1 118 426] oder ausgehend von 1,2,4-Triazoliumsalzen durch Umsetzung von 1,3,4-Oxadiazoliumsalzen mit primären Aminen (G.V. Boyd, J. Chem. Soc. C, 409, 1971).

Diese Methoden sind jedoch verhältnismäßig kompliziert, eignen sich daher nur bedingt für die Übertragung in den technischen Maßstab und haben insoweit den Nachteil, daß sie jeweils nur in bestimmten Fällen mit befriedigendem Erfolg anwendbar sind.

Der Erfindung lag daher die Aufgabe zugrunde, eine einfache, universell geeignete Synthese zur Herstellung der Triazoliumsalze Ia aufzufinden. Außerdem war es Aufgabe der Erfindung, die Triazoline Ib auf insgesamt wirtschaftlichere Weise zugänglich zu machen als bisher.

Demgemäß wurde ein Verfahren zur Herstellung der eingangs definierten Triazoliumsalze Ia und Triazoline Ib gefunden, welches dadurch gekennzeichnet ist, daß man
a) Zur Herstellung von Ia ein Amidrazon der allgemeinen Formel II 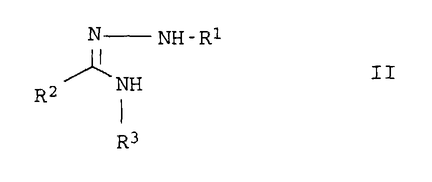 mit einer Carbonsäure der allgemeinen Formel III

   R⁴-COOH III

   oder einem funktionellen Derivat (IIIa) dieser Säure umsetzt, wobei sich aus III bzw. IIIa ein Anion A bildet, das man gewünschtenfalls durch ein anderes Anion ersetzen kann, und daß man
b) zur Herstellung von Ib die Verbindung Ia, ohne sie aus ihrem Reaktionsgemisch zu isolieren, mit einer Verbindung der allgemeinen Formel IV

   X-Y-R⁵ IV

   umsetzt, in der X Wasserstoff, ein Alkalimetallatom oder das Äquivalent eines Erdalkalimetallatoms bedeutet.

Die Amidrazone II sind z.T. bekannt oder in an sich bekannter Weise erhältlich. Besonders empfiehlt es sich, zunächst aus einem Carbonsäurechlorid R²-CO-Cl (VI) und einem primären Amin R³-NH₂ (VII) das entsprechende Carbonsäureamid (VIII) herzustellen, wie es z.B. in P. Nuhn et al., Pharmazie 48, 340 (1993) und D.G. Neilson et al. Chem. Rev. 70, 151 (1970) näher beschrieben ist, und mit einem Chlorierungsmittel, wie Phosphorpentachlorid oder Thionylchlorid, in das Imidoylchlorid (IX) zu überführen, das mit einem Hydrazin (X) zu dem gewünschten Amidrazon (II) umgesetzt wird.

Der Reaktionsweg zu den Amidrazonen (II) läßt sich somit wie folgt veranschaulichen:

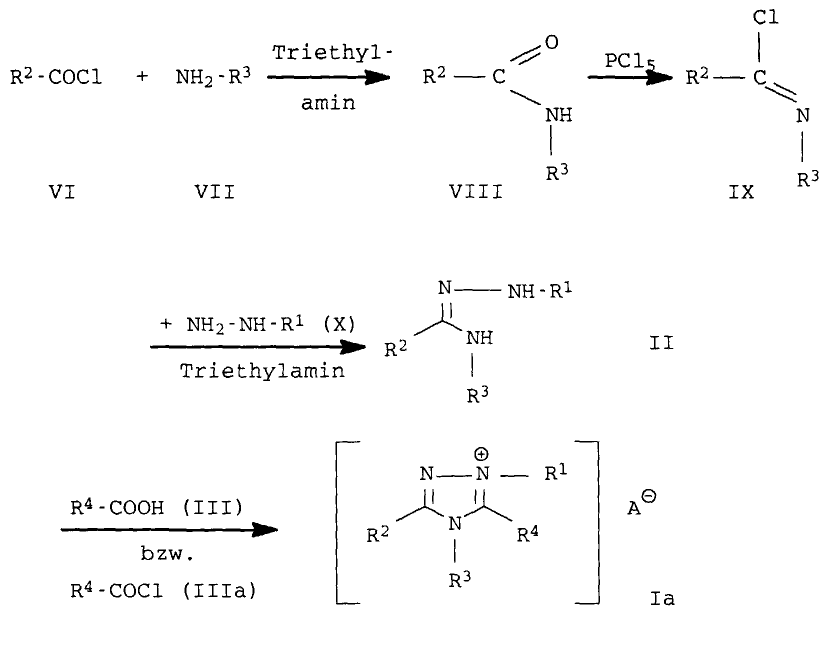

Unerwarteterweise wurde gefunden, daß man die Zwischenstufen VIII und IX dieser Reaktionsfolge nicht aus ihrem Reaktionsgemisch zu isolieren braucht, wodurch sich das Gesamtverfahren, ausgehend von VI, VII und X zu Ia bzw. Ib erheblich vereinfacht.

Zur Herstellung der Carbonsäureamide empfiehlt sich die Mitverwendung eines inerten Lösungsmittel wie Toluol, Xylol, Dimethylformamid oder Dimethylsulfoxid. Der freiwerdende Halogenwasserstoff kann entweder direkt abgeführt oder durch eine tertiäre Stickstoffbase wie Triethylamin, Pyridin oder N-Methyl-pyrrolidon gebunden werden. Vorzugsweise arbeitet man bei -20°C bis 150°C, besonders bei 0°C bis 100°C.

Die Carbonsäureamide werden sodann mit einem geeigneten Chlorierungsmittel wie Thionylchlorid oder Phosphorpentachlorid in einem der o.g. inerten Lösungsmittel zu den Imidoylchloriden umgesetzt.Vorzugsweise arbeitet man bei -20°C bis 100°C, besonders bei 0°C bis 80°C.

Im Anschluß daran setzt man die eingeengte Reaktionslösung in Gegenwart einer basischen Komponente wie Triethylamin zum Abfangen des entstehenden Halogenwasserstoffes und Hydrazin zu den gewünschten Amidrazonen (II) um, und zwar zweckmäßigerweise in einem inerten Lösungsmittel wie Tetrahydofuran, Dioxan oder Dimethylsulfoxid. Vorzugsweise arbeitet man bei -20°C bis 100°C, besonders bei 0°C bis 80°C.

Nach beendeter Reaktion isoliert man die Amidrazone zweckmäßigerweise durch Kristallisation, indem man die eingeengte Lösung mit einer wäßrigen Säure wie Essigsäure behandelt.

Nach den bisherigen Beobachtungen ist das gute Gelingen dieses Verfahrens zur Herstellung der Amidrazone II weitgehend unabhängig von der Natur der Reste R¹ bis R³.

Diese Reste können daher im Prinzip beliebige organische Reste sein, jedoch werden im Hinblick auf die Eigenschaften von Ia bzw. Ib folgende Reste bevorzugt:
- gesättigte und ungesättigte aliphatische Reste mit 1 bis 30 C-Atomen, wie Methyl, Ethyl, Propyl, Isopropyl, Butyl, Isobutyl, tert-Butyl, sec. Butyl, Pentyl, Neopentyl, n-Hexyl, n-Dodecyl und Stearyl, Vinyl und Allyl, sowie Ethinyl und Propinyl
- gesättigte und ungesättigte cycloaliphatische Reste mit 5 bis 7 Ringgliedern, wie Cyclopentyl, Cyclopentenyl, Cyclohexyl und Cyclohexenyl
- araliphatische Reste mit 7 bis 30 C-Atomen, wie Benzyl, 2-Phenylethyl, 1- und 2-Naphthylmethyl und die Phenanthrylmethyle
- iso- und heterocyclische aromatische Reste, wie Furanyl, Thienyl, Pyrrolyl, Imidazolinyl, Pyridinyl, Chinolinyl und Acridinyl, Phenyl, Naphthyl, Anthryl und Phenanthryl.

R² und R³ können auch miteinander verbunden sein, so daß es sich in diesem Falle um Lactame handelt, die man ebenso wie andere einfache Amide (VIII) unmittelbar in die Synthese einsetzen kann. Solche Lactame sind z.B. 2-Pyrazinon, 2-Pyridinon, 2-Pyrrolidon. Alle Reste R¹ bis R³ können ihrerseits Substituenten tragen, z.B. Fluor, Chlor, Brom, Cyan, Nitro und Hydroxyl, oder sie können durch Heteroatome wie Sauerstoff unterbrochen sein.

Zur Herstellung von Ia setzt man II erfindungsgemäß vorzugsweise mit einer Carbonsäure R⁴-COOH um, wobei man unmittelbar das Triazoliumsalz der Säure erhält:

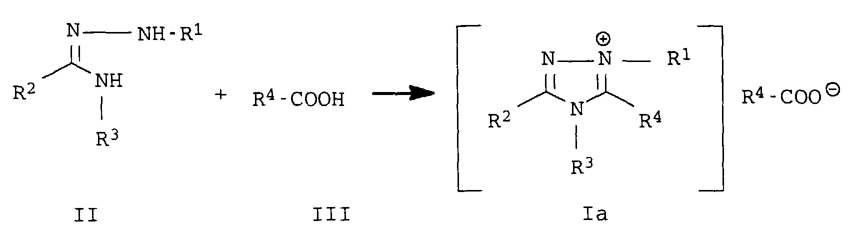

Prinzipiell kommen als Reste R⁴ Wasserstoff oder beliebige C-organische Reste, insbesondere wie sie für die Reste R¹ bis R³ genannt wurden, in Betracht. Im Hinblick auf die Eigenschaften von Ib werden als Reste R⁴ jedoch Wasserstoff und Hydroxymethyl bevorzugt, d.h. in diesen Fällen dienen Ameisensäure bzw. Glycolsäure zur Ringbildung.

Anstelle der freien Carbonsäuren III kann man auch deren funktionellen Derivate IIIa, z.B. die Carbonsäurechloride, -ester, -amide und -anhydride einsetzen.

Geht man von Chloriden aus, wird bei der Ringschlußreaktion 1 mol Chlorwasserstoff abgespalten, wobei man unmittelbar das Triazoliumchlorid (A = Cl) erhält. Analog verhält es sich bei Anhydriden. Werden die Ester (IIIa) als Reaktionskomponenten ausgewählt, setzt man zusätzlich eine Mineralsäure HA wie Chlorwasserstoff oder Perchlorsäure zu und sorgt für die laufende Entfernung des entstehenden Alkohols aus dem Reaktionsgemisch, z.B. durch azeotrope Destillation.

In einer bevorzugten Ausführungsform nimmt man die Ringschlußreaktion mit Anhydriden IIIa vor oder, falls man eine Säure III oder eines ihrer funktionellen Derivate einsetzt, in Gegenwart eines Anhydrides einer anderen Carbonsäure, die sich bei der Ringschlußreaktion im Vergleich zur Carbonsäure R⁴-COOH weniger reaktiv verhält. So empfiehlt sich im Falle von Ameisensäure als Säure III die Mitverwendung von Essigsäureanhydrid in etwa äquimolaren Mengen und im Falle von Essigsäure die Mitverwendung von beispielsweise Propionsäureanhydrid.

Vorzugsweise nimmt man die Umsetzung von II mit III bzw. IIIa in einem inerten Lösungsmittel wie Tetrahydrofuran, Dioxan oder Dimethylsulfoxid bei Temperaturen von 0°C bis 120°C vor.

Hierbei ist es zweckmäßig, eine Lösung von III bzw. IIIa vorzulegen und hierzu im Laufe der fortschreitenden Reaktion II zuzugeben. Aus der eingeengten Reaktionslösung kann Ia durch Kristallisation erhalten werden. Diese Salze bilden sich auf besonders einfache Weise, indem man mit einer Säure HA' versetzt, deren Anionen zu schwerlöslichen Salzen führen.

Anionen A' solcher Säuren sind beispielsweise Acetat, Chlorid, Bromid sowie besonders Perchlorat.

Möchte man hingegen die Triazoline Ib herstellen, isoliert man die Triazoliumsalze Ia nicht aus ihrem Reaktionsgemisch, sondern setzt sie unmittelbar mit einer Verbindung der Formel IV um.

Auch bei dieser Reaktion spielt die Natur des Restes R⁵ grundsätzlich keine Rolle, so daß hier die allgemeinen Empfehlungen für die Art der Reste R¹ bis R³ gelten.

Im Hinblick auf die hauptsächlich erwünschten Triazoline Ib sind jedoch Wasserstoff, Hydroxymethyl oder 1,2-Dihydroxyethyl als Rest R⁴ bevorzugt.

Y steht allgemein vorzugsweise für Sauerstoff und unter den Resten X werden Wasserstoff , Natrium und Kalium bevorzugt.

Die Triazoline Ib und damit auch deren Vorstufen Ia haben große technische Bedeutung als Katalysatoren für die Herstellung von Acyloinen aus Aldehyden. Näheres ist z.B. der EP-A 587 044 zu entnehmen.

### Beispiel 1

### Herstellung von N¹,N³-Diphenylbenzamidrazon

Eine Suspension aus 140,6 g (1 mol) Benzoylchlorid und 1000 ml Toluol wurde bei 5°C im Laufe von 90 min mit 93,1 g (1 mol) Anilin versetzt und danach 12 h unter Rückfluß erhitzt, wobei der gebildete Chlorwasserstoff abgezogen wurde. Zu der so erhaltenen Lösung wurden bei 25°C 356,9 g (3 mol) Thionylchlorid gegeben und anschließend wurde dieses Gemisch unter Rühren 7 h bei 80°C gehalten, wonach das überschüssige Thionylchlorid sowie das Toluol bei 1 mbar abdestilliert wurden.

Der Rückstand wurde in 1000 ml Tetrahydrofuran (THF) aufgenommen, mit 151,8 g (1,5 mol) Triethylamin und danach bei 5°C im Laufe von 1 h mit 108,1 g (1 mol) Phenylhydrazin versetzt.

Diese Mischung wurde 12h bei 20°C gerührt, eingeengt und mit 1800 ml 2%iger Essigsäure versetzt, wobei das Diphenylbenzamidrazon als hellgelber Niederschlag ausfiel, welcher abfiltriert und mit Methanol und Wasser gewaschen wurde.

Die Ausbeute an diesem Produkt (Fp =176°C) betrug 60 %.

### Beispiel 2

### Herstellung von 5-Methoxy-1,3,4-triphenyl-1,2,4-(5H)-triazolin mit Ameisensäure/Acetanhydrid

Zu einer Mischung aus 1100 g Essigsäureanhydrid und 630 g Ameisensäure wurden 220 g (0,75 mol) N¹,N³-Diphenylbenzamidrazon gegeben, wonach das Glycerid bei 25°C 12 h gerührt wurde. Nach Abziehen des Lösungsmittels verblieben 346 g eines gelben zähen Harzes. Nach Lösen des Harzes in 1000 ml Methanol wurden unter Eiskühlung 750 ml (4 mol) 30%ige Natriummethylat-Lösung zugegeben und es wurde weitere 2 h gerührt. Der Feststoff wurde abgesaugt, mit Methanol gewaschen und aus Methanol umkristallisiert. Man erhielt das 5-Methoxy-1,3,4-triphenyl-1,2,4-(5H)-triazolin in 74 %iger Ausbeute als hellgelben, kristallinen Feststoff (Fp: 114°C).

### Beispiel 3

### Herstellung von 5-Methoxy-1,3,4-triphenyl-1,2,4-(5H)-triazolin mit Ameisensäure

In 373 g (8,10 mol) Ameisensäure wurden 148 g (0,51 mol) N¹,N³-Diphenylbenzamidrazon gelöst und die Mischung wurde unter Rückfluß 16 h gerührt. Nach Abziehen der überschüssigen Ameisensäure unter vermindertem Druck bei 50°C wurden 500 ml Toluol zugegeben und die restlichen Mengen an Ameisensäure azeotrop abdestilliert. Das verbleibende gelbe, zähe Harz wurde in 1000 ml Methanol gelöst. Unter Eiskühlung wurden zu dieser Mischung 266 g (1,48 mol) 30%ige Natriummethylatlösung gegeben, wonach 1 h gerührt wurde. Der Feststoff wurde filtriert, die Mutterlauge eingeengt und der dabei ausfallende Niederschlag mit der Hauptmenge vereinigt. Nach Umkristallisation aus Methanol erhielt man das 5-Methoxy-1,3,4-triphenyl-1,2,4-(5H)-triazolin als hellgelben, kristallinen Feststoff in 61 %iger Ausbeute (Fp.: 114°C).

### Beispiel 4

### Herstellung von 1,3,4-Triphenyl-1,2,4-triazoliumperchlorat mit Ameisensäure/Acetanhydrid

Zu einer Mischung aus 43 g Essigsäureanhydrid und 24 g Ameisensäure wurden 10 g (35 mmol) N¹,N³-Diphenylbenzamidrazon gegeben und bei 25°C 12 h gerührt. Nach Abziehen der Lösungsmittel bei 50°C/30 mbar wurde der honigfarbene Rückstand mit 35 ml Perchlorsäure versetzt und 1 h nachgerührt. Nach Zugabe von 100 ml Wasser wurde der Feststoff abfiltriert und mit Wasser und Methanol gewaschen. Man erhielt das 1,3,4-Triphenyl-1,2,4-triazoliumperchlorat als hellgelben Feststoff in 80 %iger Ausbeute (Fp.: 225°C).

### Beispiel 5

### Herstellung von 1,3,4-Triphenyl-1,2,4-triazoliumperchlorat mit Ameisensäure

Eine Mischung aus 21 g Essigsäure, 24 g Ameisensäure und 10 g (35 mmol) N¹,N³-Diphenylbenzamidrazon wurde bei 110°C 12 h gerührt. Nach Abziehen der Lösungsmittel bei 50°C/30mbar wurde der viskose Rückstand mit 35 ml Perchlorsäure versetzt und 1 h nachgerührt und analog Beispiel 4 aufgearbeitet. Man erhielt das 1,3,4-Triphenyl-1,2,4-triazoliumperchlorat als hellgelben Feststoff in 69 %iger Ausbeute (Fp.: 225°C).

### Beispiel 6

### Herstellung von 1,3-Diphenyl-4-(4-nitrophenyl)-1,2,4-triazoliumperchlorat

Eine Mischung aus 43 g Essigsäureanhydrid, 24 g Ameisensäure und 10 g (30 mmol) N¹-Phenyl-N³-(4-nitrophenyl)-benzamidrazon wurde zunächst 12 h bei 25°C und danach 2 h unter Rückflußkühlung gerührt. Die Aufarbeitung analog Beispiel 4 lieferte das 1,3-Diphenyl-4-(4-nitrophenyl)-1,2,4-triazoliumperchlorat als farblose Kristalle in 75 %iger Ausbeute (Fp.: 305°C).

### Beispiel 7

### Herstellung von 1,4-Bis-(2,6-dimethylphenyl)-3-phenyl-1,2,4-triazoliumperchlorat

Zu einer Lösung aus 110 mmol (2,6-Dimethylphenyl)-hydrazin und 250 mmol Triethylamin in 150 ml THF wurde bei 25°C langsam eine Lösung von 100 mmol N-(2,6-Dimethylphenyl)-benzimidoylchlorid in 75 ml THF getropft. Nach 2,5 Stunden Rühren bei 25°C wurde das Lösungsmittel entfernt, der ölige Rückstand in Dichlormethan aufgenommen, das ungelöste Triethylammoniumhydrochlorid abfiltriert und die Dichlormethanlösung mit Wasser gewaschen. Nach Trocknen über Natriumsulfat wurde das Lösungsmittel abgezogen. Zu 50 mmol dieses Amidrazonrohproduktes wurde eine Mischung von 180 mmol Acetanhydrid und 225 mmol Ameisensäure zugegeben. Die Lösung wurde 20 h bei 100°C gerührt, die überschüssige Ameisensäure bzw. das Acetanhydrid abdestilliert und der Rückstand wurde mit 20 ml 35% Perchlorsäure 12 h gerührt. Der ausgefallene Feststoff wurde abgesaugt und mit Wasser, 1-Butanol und Petrolether gewaschen. Man erhielt 1,4-Bis-(2,6-dimethylphenyl)-3-phenyl-1,2,4-triazoliumperchlorat als farbloses Pulver in 64 %iger Ausbeute (Fp.: 271°C).

### Beispiel 8

### Herstellung von 1,4-Bis (3,5-dichlorphenyl)-3-phenyl-1,2,4-triazoliumperchlorat

Zu einer Lösung aus 110 mmol (3,5-Dichlorphenyl)-hydrazin und 250 mmol Triethylamin in 150 ml THF wurde bei 20°C langsam eine Lösung von 100 mmol N-(3,5-Dichlorphenyl)-benzimidoylchlorid in 75 ml THF getropft. Nach 2,5 Stunden Rühren bei 20°C wurde das Lösungsmittel entfernt, der ölige Rückstand in Dichlormethan aufgenommen, das ungelöste Triethylammoniumhydrochlorid abfiltriert und die Dichlormethanlösung mit Wasser gewaschen. Die weitere Umsetzung erfolgte analog Beispiel 7. Man erhielt das 1,4-Bis-(3,5-dichlorphenyl)-3-phenyl-1,2,4-triazoliumperchlorat als farbloses Pulver in 48 %iger Ausbeute (Fp.: 245°C).

## Patentansprüche

1. Verfahren zur Herstellung von 1,2,4-Triazoliumsalzen und 1,2,4-Triazolinen der allgemeinen Formel Ia bzw. Ib 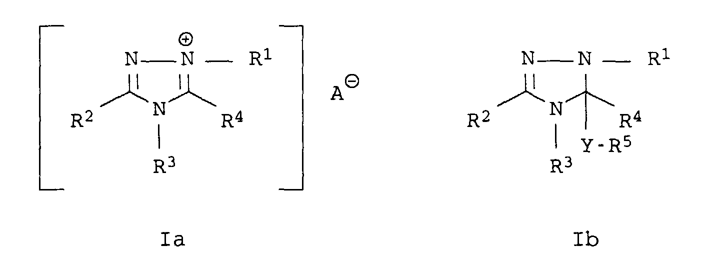 in der die Substituenten folgende Bedeutung haben:
R¹, R², R³, R⁴, R⁵ gesättigte und ungesättigte aliphatische Reste mit 1 bis 30 C-Atomen, gesättigte und ungesättigte cycloaliphatische Reste mit 5 bis 7 Ringgliedern, araliphatische Reste mit 7 bis 30 C-Atomen, iso- und heterocyclische aromatische Reste, wobei R² und R³ zu einem 5 bis 8-gliedrigen Ring verbunden sein können und alle Reste R¹ bis R⁵ ihrerseits Fluor, Chlor, Brom, Cyan, Nitro und Hydroxyl als Substituenten tragen und durch Heteroatome unterbrochen sein können
R⁴ zusätzlich Wasserstoff
A das Äquivalent eines Anions
Y Sauerstoff oder Schwefel
dadurch gekennzeichnet, daß man
a) zur Herstellung von Ia ein Amidrazon der allgemeinen Formel II 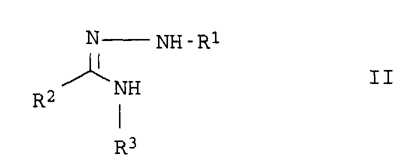 mit einer Carbonsäure der allgemeinen Formel III
R⁴-COOH III
oder einem funktionellen Derivat (IIIa) dieser Säure umsetzt, wobei sich aus III bzw. IIIa ein Anion A bildet, das man gewünschtenfalls durch ein anderes Anion ersetzen kann, und daß man
b) zur Herstellung von Ib die Verbindung Ia, ohne sie aus ihrem Reaktionsgemisch zu isolieren, mit einer Verbindung der allgemeinen Formel IV
X-Y-R⁵ IV
umsetzt, in der X Wasserstoff, ein Alkalimetallatom oder das Äquivalent eines Erdalkalimetallatoms bedeutet.

2. Verfahren zur Herstellung der Verbindungen Ia nach Anspruch 1, dadurch gekennzeichnet, daß man die Umsetzung von II mit III bzw. IIIa in Gegenwart eines Carbonsäureanhydrids (V) vornimmt, welches sich bei der Ringbildungsreaktion weniger reaktiv verhält als die Verbindung III bzw. IIIa.

3. Verfahren nach Anspruch 1 oder 2, dadurch gekennzeichnet, daß man es auf die Herstellung solcher Verbindungen Ia und Ib anwendet, in denen der Rest R⁴ Wasserstoff, die Hydroxymethyllengruppe oder die 1,2-Dihydroxyethylengruppe bedeutet.

4. Verfahren zur Herstellung der Amidrazone II gemäß Anspruch 1, dadurch gekennzeichnet, daß man ein Säurechlorid der allgemeinen Formel VI
R²-COCl VI
in einem inerten organischen Lösungsmittel mit einem Amin der allgemeinen Formel VII
NH₂-R³ VII
zu dem Amid VIII
R²-CO-NH-R³ VIII
umsetzt, und dieses ohne zu isolieren mit einem Chlorierungsmittel in das Imidoylchlorid IX 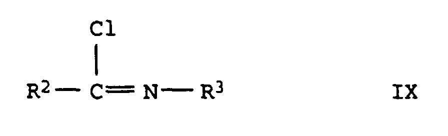 überführt, IX nach Entfernung des überschüssigen Chlorierungsmittels mit einem Hydrazin X
R¹-NH-NH₂ X
und einer tertiären Stickstoffverbindung zu II umsetzt, und II in an sich bekannter Weise aus dem Reaktionsgemisch isoliert.

## Claims

1. A process for preparing 1,2,4-triazolium salts and 1,2,4-triazolines of the general formula Ia or Ib 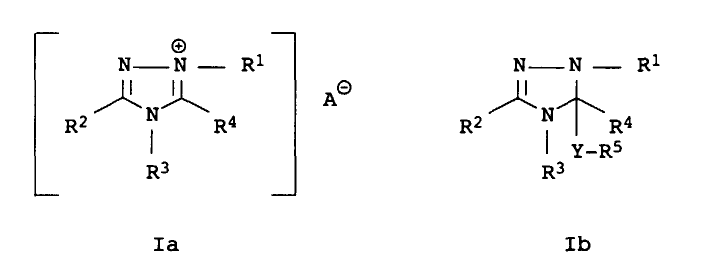 where the substituents have the following meanings:
R¹, R², R³, R⁴ and R⁵ are saturated and unsaturated aliphatic radicals having 1 to 30 C atoms,
saturated and unsaturated cycloaliphatic radicals having 5 to 7 ring members,
araliphatic radicals having 7 to 30 C atoms, iso- and heterocyclic aromatic radicals, it being possible for R² and R³ to be connected to give a 5- to 8-membered ring, and all the radicals R² to R⁵ can in turn carry fluorine, chlorine, bromine, cyano, nitro and hydroxyl as substituents and can be interrupted by heteroatoms,
R⁴ is additionally hydrogen,
A is the equivalent of an anion and
Y is oxygen or sulfur
which comprises
a) for the preparation of Ia, reacting an amidrazone of the general formula II 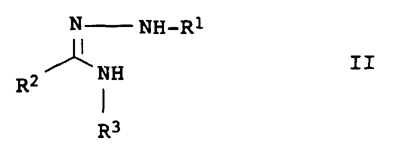 with a carboxylic acid of the general formula III
R⁴-COOH III
or a functional derivative (IIIa) of this acid, an anion A being formed from III or IIIa which, if desired, can be replaced by another anion, and
b) for the preparation of Ib, reacting the compound Ia, without isolating it from its reaction mixture, with a compound of the general formula IV
X-Y-R⁵ IV
where X is hydrogen, an alkali metal atom or an equivalent of an alkaline earth metal atom.

2. A process for preparing the compounds Ia as claimed in claim 1, wherein the reaction of II with III or IIIa is performed in the presence of a carboxylic anhydride (V) which behaves less reactively in the ring formation reaction than the compound III or IIIa.

3. A process as claimed in claim 1 or 2, wherein it is used for the preparation of those compounds Ia and Ib in which the radical R⁴ is hydrogen, the hydroxymethylene group or the 1,2-dihydroxyethylene group.

4. A process for preparing the amidrazones II as claimed in claim 1, wherein an acid chloride of the general formula VI
R²-COCl VI
is reacted in an inert organic solvent with an amine of the general formula VII
NH₂-R³ VII
to give the amide VIII
R²-CO-NH-R³ VIII
and this is converted without isolation, using a chlorinating agent, into the imidoyl chloride IX 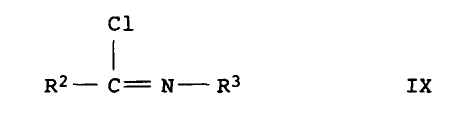 IX is reacted, after removal of the excess chlorinating agent, with a hydrazine X
R¹-NH-NH₂ X
and a tertiary nitrogen compound to give II, and II is isolated from the reaction mixture in a manner known per se.

## Revendications

1. Procédé de préparation de sels de 1,2,4-triazolium et de 1,2,4-triazolines de formules générales Ia ou Ib 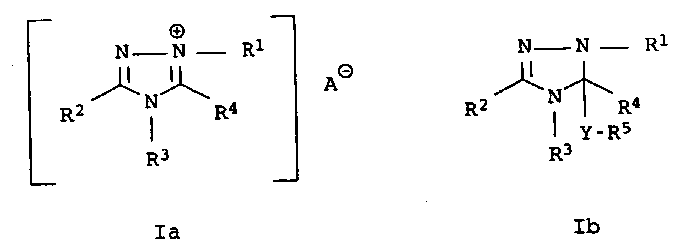 dans lesquelles les substituants prennent la signification suivante :
R¹, R², R³, R⁴, R⁵ représentent des restes aliphatiques saturés et insaturés à 1-30 atomes de C,
des restes cycloaliphatiques saturés et insaturés à 5-7 maillons dans le cycle,
des restes araliphatiques à 7-30 atomes de C, des restes aromatiques iso- et hétérocycliques, où R² et R³ peuvent être reliés en un cycle à 5-8 maillons et tous les restes R¹ à R⁵ peuvent porter des atomes de fluor, de chlore, de brome, des groupements cyano, nitro et hydroxyle en tant que substituants et peuvent être interrompus par des hétéroatomes,
R⁴ peut représenter en outre un atome d'hydrogène,
A représente l'équivalent d'un anion,
Y représente un atome d'oxygène ou de soufre,
caractérisé en ce que
a) afin de préparer la, on fait réagir une amidrazone de formule générale ^{II} 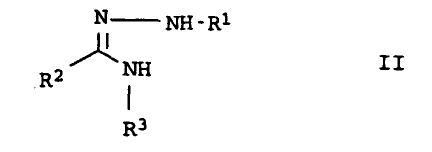 avec un acide carboxylique de formule générale III
R⁴-COOH III
ou avec un dérivé fonctionnel (IIIa) de cet acide, ce qui entraîne la formation d'un anion A à partir de III ou IIIa, que l'on peut éventuellement remplacer par un autre anion, et en ce que
b) afin de préparer Ib, on fait réagir le composé la, sans l'isoler du mélange réactionnel, avec un composé de formule générale IV
X-Y-R⁵ IV
dans laquelle X représente un atome d'hydrogène, un atome de métal alcalin ou l'équivalent d'un atome de métal alcalinoterreux.

2. Procédé de préparation de composés la selon la revendication 1, caractérisé en ce que l'on effectue la réaction de II avec III ou IIIa en présence d'un anhydride d'acide carboxylique (V), qui lors de la réaction de formation du cycle est moins réactif que les composants III ou IIIa.

3. Procédé selon la revendication 1 ou 2, caractérisé en ce que l'on utilise pour la préparation de composés Ia ou Ib dans lesquels le reste R⁴ représente un atome d'hydrogène, un groupement hydroryméthyléne ou un groupement 1,2-dihydroxyéthylène.

4. Procédé de préparation de l'amidrazone II selon la revendication 1, caractérisé en ce que l'on fait réagir un chlorure d'acide de formule générale VI
R²-COCl VI
dans un solvant organique inerte avec une amine de formule générale VII
NH₂-R³ VII
pour donner un amide VIII
R²-CO-NH-R³ VIII
que l'on transforme, sans l'isoler, avec un agent de chloration en le chlorure d'imidoyle IX 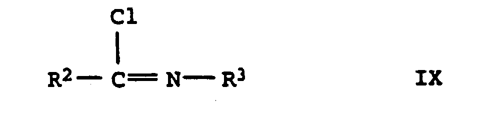 on fait réagir IX, après élimination de l'agent de chloration en excès, avec une hydrazine X
R¹-NH-NH₂ X
et un composé azoté tertiaire pour donner II, puis on soie II du mélange réactionnel, d'une façon connue en soi.
